(19) Europäisches Patentamt
European Patent Office
Office européen des brevets

(11) Veröffentlichungsnummer: **0 125 592**
**B1**

(12) # EUROPÄISCHE PATENTSCHRIFT

(45) Veröffentlichungstag der Patentschrift: **23.07.86**

(21) Anmeldenummer: **84105105.5**

(22) Anmeldetag: **05.05.84**

(51) Int. Cl.[4]: **C 10 M 131/00**, C 10 M 133/00, C 10 M 135/00, C 10 M 137/00, C 10 M 139/02

(54) **Verwendung von Tri- und Tetrachlorbutanolderivaten als Schmiermittelzusatz sowie Schmiermittel, enthaltend solche Zusätze.**

(30) Priorität: **13.05.83 DE 3317355**

(43) Veröffentlichungstag der Anmeldung: **21.11.84 Patentblatt 84/47**

(45) Bekanntmachung des Hinweises auf die Patenterteilung: **23.07.86 Patentblatt 86/30**

(84) Benannte Vertragsstaaten: **AT BE DE FR GB IT SE**

(56) Entgegenhaltungen:
**EP-A-0 004 957**
**DE-B-1 041 624**
**DE-C-952 925**
**US-A-2 897 152**
**US-A-4 207 272**
**US-A-4 247 489**
**US-A-4 289 712**

(73) Patentinhaber: **BASF Aktiengesellschaft, Carl-Bosch- Strasse 38, D-6700 Ludwigshafen (DE)**

(72) Erfinder: **Greif, Norbert, Dr., Im Woogtal 3, D-6719 Bobenheim (DE)**
Erfinder: **Oppenlaender, Knut, Dr., Otto- Dill- Strasse 23, D-6700 Ludwigshafen (DE)**
Erfinder: **Stork, Karl, Dr., Reutersgarten 1, D-6840 Lampertheim (DE)**
Erfinder: **Strickler, Rainer, Dr., Erwin- Rohde- Strasse 20, D-6900 Heidelberg (DE)**
Erfinder: **Vogel, Hans- Henning, Dr., Hans- Purrmann- Strasse 7c, D-6710 Frankenthal (DE)**
Erfinder: **Starke, Klaus, Halbergstrasse 5, D-6719 Weisenheim (DE)**
Erfinder: **Schmidt, Helmut, Thomas- Mann- Strasse 112, D-6700 Ludwigshafen (DE)**

EP 0 125 592 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft die Verwendung von 4,4,4-Tri- und/oder 2,4,4,4 - Tetrachlorbutanolderivaten, als Schmiermittelzusatz, sowie Schmiermittel, die solche Zusätze enthalten.

Die Schmierung bewegter Maschinenteile, besonders solcher, die extremen Beanspruchungen ausgesetzt sind, spielt bei der Eindämmung von Reibung und Reibungsverschleiß eine entscheidende Rolle.

Aus vielen Veröffentlichungen ist bekannt, daß durch Zusatz von Hochdruckadditiven zu Schmiermitteln deren Schmiereigenschaften, speziell im Bereich der Grenzschmierung, verbessert werden können.

Häufig werden dazu Phosphor-, Schwefel- oder Chlorverbindungen verwendet. Bei den Chlorverbindungen kommt dabei den Chlorparaffinen große Bedeutung zu (J. Falbe, U. Hasserodt "Katalysatoren, Tenside und Mineralöl-additive", S. 247 f., G. Thieme Verlag, Stuttgart (1978) u. dort zitierte Literatur).

Auch polychlorierte Carbonsäuren sind als Hochdruckadditive vorgeschlagen worden. So werden in den DE-Patentschriften 952 925 und 1 041 624 besonders 10,12,12,12 - Tetrachlorlaurinsäurederivate erwähnt.

Nach dem Vorschlag der DE-Auslegeschrift 1 033 355 bzw. FR-Patentschrift 1 242 382 können 2,2,4,4 - Tetrachlorbuttersäure bzw. deren Niederalkylester als Zusatz für Schmiermittel verwendet werden.

Derivate, die sich von Buttersäuren mit einer endständigen Trichlormethylgruppe ableiten, sind in der EP—A—4 957 für diesen Zweck beschrieben.

In den US-Patentschriften 4 207 272, 4 247 489 und 4 289 712 werden 2,4,4,4 - Tetrachlorbutyl- bzw. 1,4,4,4 - Tetrachlorbutylphosphite beschrieben, wobei u.a. auch ihre Verwendung als Schmiermittelzusatz erwähnte wird.

Es wurde nun gefunden, daß 4,4,4-Tri- und/oder 2,4,4,4 - Tetrachlorbutanolderivate der allgemeinen Formel (I)

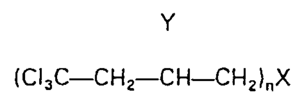 I

in der Y ein Chloratom oder eine Hydroxylgruppe, n eine Zahl von 1 bis 4 und X für n=1 einen der Reste

$—OR^1$, 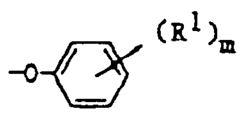 $—NHR^1$, $—NR^1_2$, 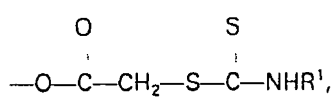

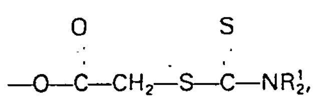 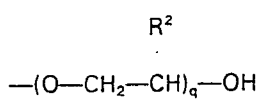 oder 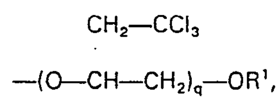

oder für n=2 einen der folgenden Reste

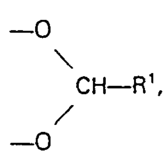 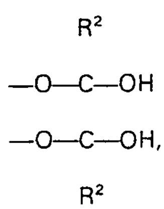 N—$R^1$, 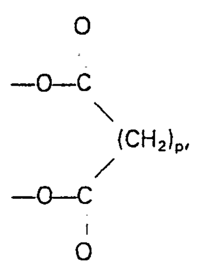 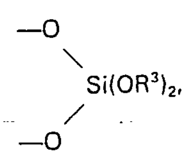 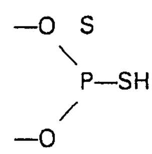

oder dessen Zn-Salz, oder für n=3 einen der Reste

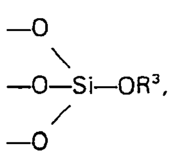 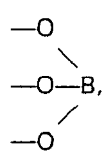 oder für n=4 den Rest 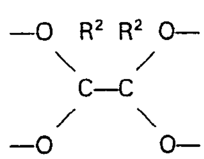

darstellt, wobei $R^1$ eine geradkettige oder verzweigte Alkylgruppe mit 1 bis 15 Kohlenstoffatomen, $R^2$ ein Wasserstoffatom, eine Methyl- oder Ethylgruppe, $R^3$ eine geradkettige oder verzweigte Alkylgruppe mit 1

bis 4 Kohlenstoffatomen, m eine Zahl von 1 bis 3, q eine Zahl von 1 bis 5 und p eine Zahl von 1 bis 4 bedeutet, vorteilhaft als Schmiermittelzusatz verwendet werden können.

Beispielsweise kann $R^1$ eine Methyl-, Ethyl-, Propyl-, Isopropyl-, Butyl-, t-Butyl-, Isoamyl-, Hexyl-, Octyl-, 2-Ethylhexyl-, Nonyl-, Decyl-, Dodecyl-, Tridecyl- oder Pentadecylgruppe sein.

Für $R^3$ kommt z.B. eine Methyl-, Ethyl-, Isopropyl-, Butyl- oder t-Butylgruppe in Betracht.

Die Synthese einiger der Verbindungen der Formel I ist beschrieben, vgl. u.a. Tetrahedron *32*, 2295 (1965); C. R. Hebd. Seance Acad. Sci. Ser. C *283*, 187, (1976); J. Chem. Eng. Data *13*, 566 (1966); US-Patentschriften 3 843 569 und 3 968 170; EP—A—22 629.

Die übrigen erfindungsgemäß zu verwendenden Produkte können alle nach an sich bekannten Methoden hergestellt werden. Insbesondere dadurch, daß man z.B. 2,4,4,4 - Tetrachlorbutanol der Formel (II)

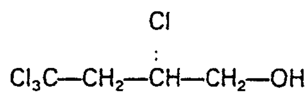

II

oder 4,4,4 - Trichlor - 1,2 - epoxybutan der Formel (III)

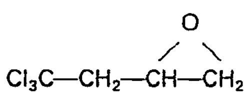

III

den üblichen Veretherungs-, Aminierungs-, Acetalisierungs- oder Veresterungsreaktionen unterwirft.

Als Reaktionspartner kommen für Veretherungsreaktionen Alkohole der Formel $R^1$—OH, Phenole der Formel

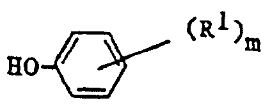

oder Epoxide der Formel

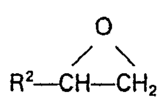

in Frage. Aminierungen lassen sich mit Aminen des Typs $R^1$—$NH_2$ oder $(R^1)_2NH$ durchführen. Für die Acetalisierungsreaktionen verwendet man zweckmäßigerweise Monoaldehyde der Formel $R^1$—CHO oder Dialdehyde, wie Glyoxal. Die Veresterungen werden üblicherweise mit den freien Säuren, gegebenenfalls unter zusätzlicher Säurekatalyse, durchgeführt. Es ist aber auch möglich, diesen Schritt durch Umesterung bzw. wie im Fall der Dithiophosphate, durch Umsetzung mit Phosphorpentasulfid vorzunehmen. Als Folgereaktionen lassen sich zusätzlich S-Alkylierungen, beispielsweise die Umsetzung von Nadithiocarbamaten mit den entsprechenden Halogenalkylverbindungen, vollziehen.

2,4,4,4-Tetrachlorbutanol (II) und 4,4,4 - Trichlor - 1,2 - epoxybutan (III) sind bekannte Verbindungen. Sie lassen sich gemäß den US—PSen 2 568 859, 3 399 217 und 3 923 844 synthetisieren. Die für die jeweiligen Umsetzungen (Veretherung, Aminierung etc.) verwendeten Reagenzien sind ebenfalls bekannt.

Die Tri- und Tetrachlorbutanolderivate der Formel I können sowohl mineralischen als auch synthetischen Schmiermitteln (z.B. Polyetheröle, Esteröle, Phosphorsäureester, silicium- oder halogenhaltige Öle) zugesetzt werden.

Schon geringe Konzentrationen dieser Stoffe im Schmiermittel, z.B. 0,05 bis 10 Gew.%, vorzugsweise 0,1 bis 5 Gew.%, bezogen auf die Gesamtmischung, reichen aus, um die Schmiereigenschaften, besonders unter Grenzschmierbedinungen, entscheidend zu verbessern.

Zusätzlich können die Schmiermittel noch mit anderen Additiven, die in der modernen Schmiertechnik Verwendung finden, ausgerüstet sein. Dabei handelt es sich um an sich bekannte Hilfsmittel, beispielsweise Oxidationsinhibitoren, Viskositäts-Index-Verbesserer, Pourpointverbesserer, Detergentien, Dispersants, Schaumverhütungsmittel, Demulgatoren oder Korrosionsinhibitoren (vgl. J. Falbe, U. Hasserodt "Katalysatoren, Tenside und Mineralöladditive", S. 235 f., G. Thieme Verlag, Stuttgart (1978) oder Ullmann "Encyklopädie der technischen Chemie" *20*, 540 f., 4. Auflage, Verlage Chemie, Weinheim (1981)).

Die folgenden Beispiele sollen die Erfindung erläutern, wobei im Teil A die Herstellung der Produkte und im Teil B ihre Anwendung beschrieben ist.

A)

Beispiel 1

4,4,4-Trichlor-2-hydroxybutyl-i-tridecylether

50 g i-Tridecanol und 0,5 g Bortrifluorid-diethyletherat wurden gemischt und unter Stickstoff auf 80°C

erwärmt. Dann tropfte man 43,9 g 4,4,4 - Trichlor - 1,2 - epoxybutan so zu, daß die Temperatur den Wert von 100°C nicht überschritt. Nach 5-stündigem Nachrühren bei 100°C war die Reaktion beendet.

Man erhielt 93.9 g 4,4,4 - Trichlor - 2 - hydroxybutyl - i - tridecylether (Additiv 1).

Beispiel 2

4,4,4-Trichlor-2-hydroxybutyl-2',4'-di-t-butylphenylether

41,2 g 2,4-Di-t-butylphenol und 0,41 g Bortrifluorid - diethyletherat wurden gemischt und unter Stickstoff auf 30°C erwärmt. Dann tropfte man 35,1 g 4,4,4 - Trichlor - 1,2 - epoxybutan so zu, daß die Temperatur den Wert von 100°C nicht überschritt. Nach 5-stündigem Nachrühren bei 100°C war die Reaktion beendet.

Man erhielt 76,3 g 4,4,4 - Trichlor - 2 - hydroxybutyl - 2',4' - di - t - butylphenylether (Additiv 2).

Beispiel 3

2-Ethylhexylpenta(2,2,2-trichlorethyl)glykol

Ein Gemisch aus 10,4 g 2-Ethylhexanol, 14,4 g 4,4,4 - Trichlor - 1,2 - epoxybutan und 4 g saure Bleicherde (R)Tonsil FF (in der Bundesrepublik eingetragenes Warenzeichen der Firma Süd-Chemie, München) wurde auf 120°C erwärmt. Nach 30 min. tropfte man weitere 57,5 g 4,4,4 - Trichlor - 1,2 - epoxybutan zu und rührte 2 Stunden bei 120°C nach. Nach diesem Zeitraum konnte kein Epoxid mehr nachgewiesen werden. Man filtrierte vom Katalysator ab und erhielt 82,3 g 2 - Ethylhexylpenta(2,2,2 - trichlorethyl)glykol) (Additiv 3).

Beispiel 4

Bis(4,4,4-trichlor-2-hydroxybutyl)tridecylamin

35 g Tridecylamin wurden auf 60°C erwärmt und tropfenweise mit 54 g 4,4,4 - Trichlor - 1,2 - epoxybutan versetzt. Danach wurde auf 100°C erhitzt und 2 Stunden nachgerührt. Nach diesem Zeitraum konnte kein Epoxid mehr nachgewiesen werden. Man erhielt 89 g Bis(4,4,4 - trichlor - 2 - hydroxybutyl)tridecylamin (Additiv 4).

Beispiel 5

(4,4,4-Trichlor-2-hydroxybutyl)bis(2-ethylhexyl)amin

48,2 g Bis(2-ethylhexyl)amin wurden unter Stickstoff auf 90°C erwärmt. Anschließend tropfte man bei 90 bis 100°C 35,1 g 4,4,4 - Trichlor - 1,2 - epoxybutan zu. Man rührte noch 5 Stunden bei 120°C nach. Nach diesem Zeitraum konnte kein Epoxid mehr nachgewiesen werden. Man erhielt 83,3 g (4,4,4 - Trichlor - 2 - hydroxybutyl)bis(2 - ethylhexyl)amin (Additiv 5).

Beispiel 6

2-Ethylhexanalbis(2,4,4,-tetrachlorbutyl)acetal

149 g 2,4,4,4 - Tetrachlorbutanol, 45 g 2-Ethylhexanal und 10 g saure Bleicherde Tonsil Ff wurden in 150 ml n-Hexan gegeben. Man erhitzte zum Rückfluß und kreiste mit Hilfe eines Wasserabscheiders 7 ml Wasser aus. Nach dem Abkühlen wurde vom Katalysator abfiltriert und das Filtrat bei 120°C und 27 mbar eingeengt. Man erhielt 177 g 2 - Ethylhexanalbis(2,4,4,4 - tetrachlorbutyl)acetal (Additiv 6).

Beispiel 7

Glyoxaltetrakis(2,4,4,4 - tetrachlorbutyl)acetal und Glyoxalbis(2,4,4,4 - tetrachlorbutyl)halbacetal

224 g 2,4,4,4-Tetrachlorbutanol, 29 g 40 %iges wäßriges Glyoxal und 24 g saure Bleicherde Tonsil FF wurden in 180 ml n-Hexan ca. 18 Stunden unter Rückfluß erhitzt. Dabei kreiste man mit Hilfe eines Wasserabscheiders 22 g Wasser aus. Nach dem Abkühlen wurde vom Katalysator abfiltriert und das Filtrat im Wasserstrahlvakuum vom Lösungsmittel befreit. Man erhielt 230 g einer Mischung aus Glyoxaltetrakis(2,4,4,4 - tetrachlorbutyl)acetal und dessen Halbacetal (Nachweis mittels IR-Spektrum) (Additiv 7).

Beispiel 8

Adipinsäurebis(2,4,4,4-tetrachlorbutyl)ester

72 g 2,4,4,4 - Tetrachlorbutanol, 29,6 g Adipinsäuredimethylester und 1 g p-Toluolsulfonsäure wurden in 115 ml Toluol zum Rückfluß erhitzt. Dabei kreiste man mit Hilfe eines Abscheiders 10,2 g Methanol aus.

Das Reaktionsgemisch wurde einer Vakuumdestillation unterworfen, um vom Toluol zu befreien (0,3 mbar; max. Innentemp. 150°C). Als Rückstand blieben 80 g Adipinsäurebis(2,4,4,4 - tetrachlorbutyl)ester (Additiv 8).

Beispiel 9

O,O'-Bis(2,4,4,4-tetrachlorbutyl)hydrogendithiophosphat

111 g Phosphorpentasulfid wurden in 500 ml Toluol gegeben und auf 80°C erhitzt. Bei dieser Temperatur tropfte man 424 g 2,4,4,4 - Tetrachlorbutanol langsam zu. Danach wurde die Mischung solange bei 110°C gerührt, bis kein Schwefelwasserstoff mehr entwickelt wurde (Dauer ca. 24 Stunden). Nach dem Abdestillieren der flüchtigen Anteile bei Temperaturen bis 150°C isolierte man 520 Teile O,O' - Bis(2,4,4,4 - tetrachlorbutyl)hydrogendithiophosphat (Additiv 9).

Beispiel 10

Zink-O,O'-bis(2,4,4,4-tetrachlorbutyl)dithiophosphat

27,5 g basisches Zinkcarbonat (2 $ZnCO_3$ . 3 $Zn(OH)_2$) wurden mit 259 g O,O' - Bis(2,4,4,4 - tetrachlorbutyl)dithiophosphat in 1000 ml Toluol 4 Stunden bei 70°C gerührt. Nach dem Abkühlen wurde im Wasserstrahlvakuum bei Temperaturen bis 50°C das Lösungsmittel abgezogen. Man erhielt 275 g Zink - O,O' - bis(2,4,4,4 - tetrachlorbutyl)dithiophosphat (Additiv 10).

Beispiel 11

(2,4,4,4-Tetrachlorbutyl)-triethyl-orthosilicat

280 g 2,4,4,4-Tetrachlorbutanol, 275 g Tetraethyl-orthosilicat und 2,8 g Natriummethylat wurden in 250 ml Toluol gegeben und unter Stickstoff 3 Stunden auf 98°C erhitzt. Danach wurde aus dem Reaktionsgemisch Ethanol und Toluol abdestilliert, wobei die Temperatur langsam auf 160°C gesteigert wurde. Der Rückstand wurde anschließend im Rotationsverdampfer noch von anhaftendem Toluol befreit. Auf diese Weise erhielt man 507 g (2,4,4,4 - Tetrachlorbutyl) - triethyl - orthosilicat (Additiv 11).

Beispiel 12

Tris(2,4,4,4-tetrachlorbutyl)-borat

95,4 g 2,4,4,4-Tetrachlorbutanol und 9,3 g Borsäure wurden bei einem Druck von 15 mbar zunächst 4 Stunden bei 85°C und anschließend 2 Stunden bei 100°C gerührt. Dabei schieden sich 8,3 Wasser ab. Als Rückstand erhielt man 96,4 g Tris(2,4,4,4 - tetrachlorbutyl) - borat (Additiv 12).

Beispiel 13

N,N-Dibutyl-dithiocarbamidsäure-S-(methylcarbonyloxy - 2,4,4,4 - tetrachlorbutyl)ester

33 g Natrium-N,N-dibutyldithiocarbamat wurden in 60 ml Dioxan gegeben und auf 80°C erwärmt. Bei dieser Temperatur wurden zunächst 35 g Chloressigsäure - 2,4,4,4 - tetrachlorbutylester zugetropft und anschließend rührte man 10 Stunden nach. Das bei der Reaktion ausgefallene Kochsalz wurde abfiltriert und mit Dioxan gewaschen. Die vereinigten organischen Filtrate wurden bei einem Druck von 30 mbar und einer Temperatur von 60°C eingeengt. Man erhielt 59 g N,N - Dibutyl - dithiocarbamidsäure - S - (methylcarbonyloxy - 2,4,4,4 - tetrachlorbutyl) - ester (Additiv 13).

B)

Die anwendungstechnischen Prüfungen wurden im Shell-Vierkugel-Apparat, in der Almen-Wieland-Maschine und in der FZG - Zahnrad - Verspannungs - Prüfmaschine vollzogen. Im einzelnen wurden die folgenden Ergebnisse erhalten:

Beispiel 14

Prüfung im Shell-Vierkugel-Apparat (DIN 51350)

Gemessen wurde die Schweißkraft (Prüfkraft, bei der ein Verschweißen des Vierkugelsystems im Prüfgerät eintritt) und der Kalottendurchmesser (unter den Prüfbedingungen entstandener mittlerer Kalottendurchmesser der drei Stahlkugeln).

Als Prüföl wurde ein Solventraffinat mit einer Viskosität von 10,5 cSt bei 100°C bzw. 92 cSt bei 40°C und einem Viskositätsindex von 95 verwendet. Die Additivkonzentration betrug jeweils 3 Gew.%, bezogen auf die verwendete Mischung.

| Additiv Nr. | Schweißkraft [N] | Kalottendurchmesser [mm] |
|---|---|---|
| ohne Additiv | 1500 | 3,7 |
| 1 | 2800 | 2,9 |
| 2 | 2600 | 2,6 |
| 3 | 3000 | 2,8 |
| 4 | 3400 | 2,1 |
| 5 | 3200 | 3,0 |
| 6 | 2800 | 2,7 |
| 7 | 3600 | 2,8 |
| 8 | 3300 | 2,7 |
| 9 | 8000 | 2,0 |
| 10 | 6500 | 1,9 |
| 11 | 2800 | 2,7 |
| 12 | 3000 | 1,4 |
| 13 | 4000 | 3,1 |

Beispiel 15

Prüfung in der Almen-Wieland-Maschine

Gemessen wurde die aufgebrachte maximale Normalkraft, sowie die sich dabei einstellende Reibkraft. Der Reibungskoeffizient $\mu_R$ ist der Quotient von Reibkraft und Normalkraft.

Als Prüföl diente das in Beispiel 14 beschriebene Schmiermittel. Die Additivkonzentration lag bei 3 Gew.%, bezogen auf die verwendete Mischung.

| Additiv Nr. | Normalkraft [N] | Riebkraft [N] | $\mu_R$ |
|---|---|---|---|
| ohne Additiv | 3000 | 1200 | 0,4 |
| 1 | 31 000 | 3500 | 0,11 |
| 2 | 31 500 | 3400 | 0,11 |
| 3 | 28 500 | 3400 | 0,12 |
| 4 | 23 500 | 3500 | 0,15 |
| 5 | 23 000 | 3500 | 0,15 |
| 6 | 27 500 | 3500 | 0,13 |
| 7 | 31 000 | 3400 | 0,11 |
| 8 | 15 500 | 3500 | 0,23 |
| 9 | 30 000 | 3300 | 0,11 |
| 10 | 30 000 | 3300 | 0,11 |
| 11 | 26 500 | 3500 | 0,13 |
| 12 | 26 500 | 3450 | 0,13 |
| 13 | 26 500 | 3500 | 0,13 |

Beispiel 16
Prüfung in der FZG-Zahnrad-Verspannungs-Prüfmaschine (DIN 51354)
Gemessen wurde die Schadens-Kraftstufe.

Als Schmiermittel wurde ein Solventraffinat (SAE 30) mit einer Dichte von 0,885 g/cm$^3$ bei 15°C, einer Viskosität von 92 cSt bei 40°C bzw. 10,5 cSt bei 100°C und einem Viskositätsindex von 95 verwendet. Der Pourpoint liegt bei −12°C.

Das Schmiermittel enthielt einen Pourpointverbesserer. Die jeweilige Additivkonzentration ist aus der Tabelle zu entnehmen.

| Additiv Nr. | Additiv-Konzentration bezogen auf die verwendete Mischung [Gew.%] | Schadens-Krafstufe |
|---|---|---|
| ohne Additiv | — | 6—7 |
| 1 | 6,5 | 12 |
| 2 | 6,5 | 12 |
| 4 | 6,5 | 12 |
| 5 | 6,5 | 8 |
| 6 | 3,0 | 9 |
| 7 | 3,0 | 12 |
| 9 | 3,0 | 12 |
| 12 | 6,5 | 10 |

Beispiel 17

Beispiel 17 wurde analog Beispiel 16 durchgeführt, jedoch wurde als Prüföl eine Getriebeölvermischung der SAE-Klasse 80 W/90 mit einer Dichte von 0,885 g/cm$^3$ bei 15°C, einer Viskosität von 155 cSt bei 40°C bzw. 15 cSt bei 100°C und einem Viskositätsindex von 95 verwendet. Der Pourpoint des Prüföls,

das keinen Pourpointverbesserer enthält, liegt bei −27°C. Die jeweilige Additivkonzentration ist aus der Tabelle zu entnehmen.

| Additiv Nr. | Additivkonzentration bezogen auf die verwendete Mischung [Gew.%] | Schadens-Kraftstufe |
|---|---|---|
| 9 | 6,5 | 12 |
| 11 | 6,5 | 12 |

**Patentansprüche**

1. Verwendung von 4,4,4-Tri- und/oder 2,4,4,4 - Tetrachlorbutanolderivaten der allgemeinen Formel (I)

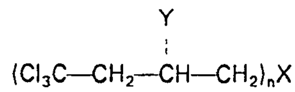 I

in der Y ein Chloratom oder eine Hydroxylgruppe, n eine Zahl von 1 bis 4 und X für n=1 einen der Reste

$—OR^1$, 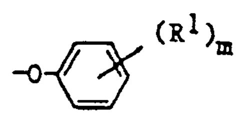 $—NHR^1$, $—NR^1_2$, 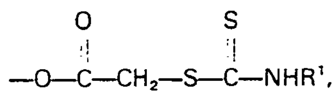

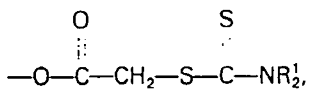 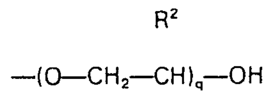 oder 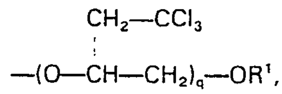

oder für n=2 einen der folgenden Reste

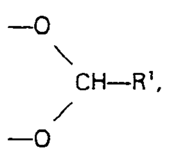 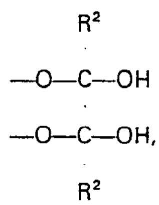 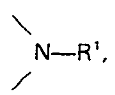 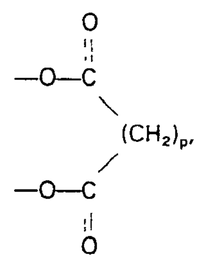 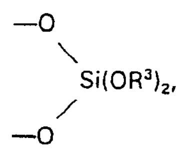 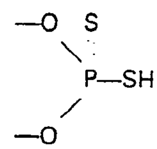

oder dessen Zn-Salz, oder für n=3 einen der Reste

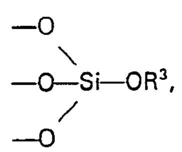 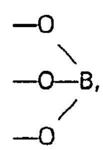 oder für n=4 den Rest 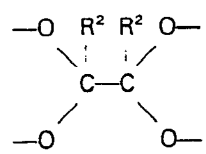

darstellt, wobei $R^1$ eine geradkettige oder verzweigte Alkylgruppe mit 1 bis 15 Kohlenstoffatomen, $R^2$ ein Wasserstoffatom, eine Methyl- oder Ethyl-gruppe, $R^3$ eine geradkettige oder verzweigte Alkylgruppe mit 1 bis 4 Kohlenstoffatomen, m eine Zahl von 1 bis 3, q eine Zahl von 1 bis 5 und p eine Zahl von 1 bis 4 bedeutet, als Schmiermittelzusatz.

2. Schmieröl mit Schmierviskosität aus der Klasse der mineralischen oder synthetischen Schmiermittel, enthaltend als Zusatz in Mengen von 0,05 bis 10 Gew.%, bezogen auf die Gesamtmischung, eine Verbindung der Formel I gemäß Anspruch 1.

**Revendications**

1. Utilisation, comme additifs pour lubrifiants, de dérivés de 4,4,4-tri- et/ou 2,4,4,4-tétrachlorobutanol de formule générale (I)

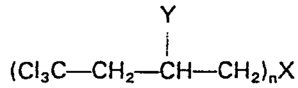 I

dans laquelle Y représente un atome de chlore ou un groupe hydroxyle, n un nombre de 1 à 4 et X représente, pour n=1, l'und es radicaux

$—OR^1$, 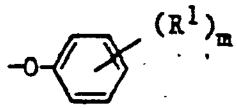 $—NHR^1$, $—NR^1_2$, 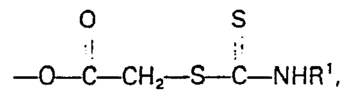

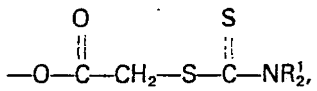 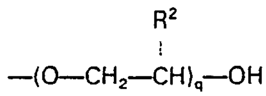 ou 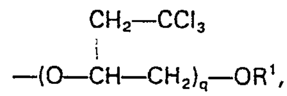

pour n=2, l'un des radicaux suivants

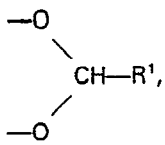 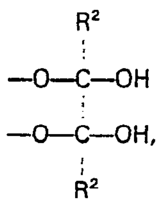 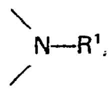 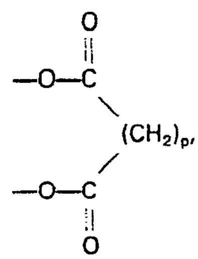 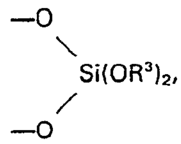 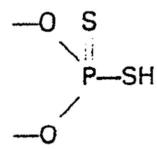

ou leurs sels de Zn, pour n=3, l'un des radicaux

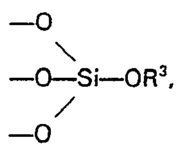 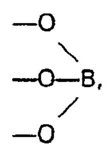 ou pour n=4, le radical 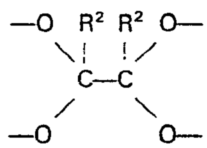

$R^1$ représentant un groupe alkyle à 1—15 atomes de carbone à chaîne droite ou ramifiée, $R^2$ un atome d'hydrogène, un groupe méthyle ou éthyle, $R^3$ un groupe alkyle à 1—4 atomes de carbone à chaîne droite ou ramifiée, m un nombre de 1 à 3, q un nombre de 1 à 5 et p un nombre de 1 à 4.

2. Huile de graissage à viscosité de lubrification de la classe des lubrifiants minéraux ou synthétiques, contenant en tant qu'additif, dans des proportions de 0,05 à 10% en poids, sur la base du mélange total, un composé de formule I selon la revendication 1.

## Claims

1. The use of a 4,4,4-trichlorobutanol and/or 2,4,4,4-tetrachlorobutanol derivative of the general formula (I)

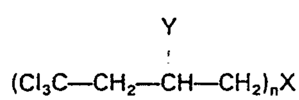 I

where Y is chlorine or hydroxyl, n is a number from 1 to 4, and X denotes, when n=1, one of the radicals

$—OR^1$, 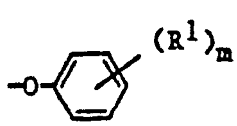 $—NHR^1$, $—NR^1_2$, 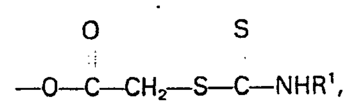

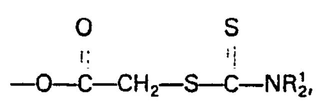 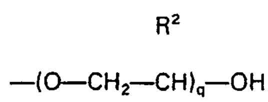 or 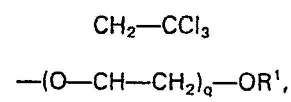

or when n=2, one of the following radicals

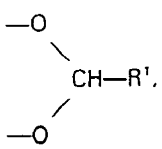 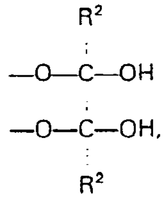 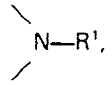 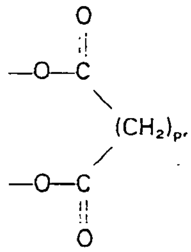 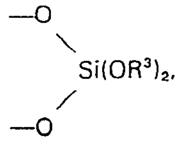 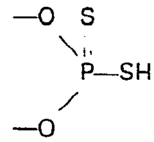

or its zinc salt, or when n=3, one of the radicals

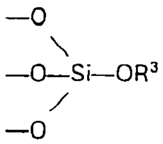 or 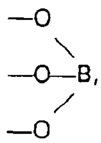 or when n=4, the radical 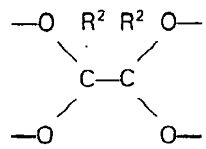

$R^1$ denoting a linear or branched alkyl of 1 to 15 carbon atoms, $R^2$ hydrogen, methyl or ethyl, $R^3$ linear or branched alkyl of 1 to 4 carbon atoms, m a number from 1 to 3, q a number from 1 to 5, and p a number from 1 to 4, as an additive for lubricants.

2. A lubricating oil exhibiting lubricity and selected from the class of mineral or synthetic lubricants, which contains, as additive, a compound of the formula I as claimed in claim 1 in an amount of from 0.05 to 10% by weight, based on the total mixture.